# EUROPÄISCHE PATENTSCHRIFT

(11) **EP 1 695 730 B1**
(45) Veröffentlichungstag und Bekanntmachung des Hinweises auf die Patenterteilung: **01.08.2007**
(21) Anmeldenummer: 06003113.5
(22) Anmeldetag: 16.02.2006
(51) Int. Cl.: A61M 16/00

(54) **Bauteil für eine Inhalationsvorrichtung und Inhalationsvorrichtung mit diesem Bauteil**
Component for an inhalation device and an inhalation device with this component
Composant pour un dispositif d'inhalation et un dispositif d'inhalation avec ce composant

(30) Priorität: 23.02.2005 EP 05003882
(43) Veröffentlichungstag der Anmeldung: 30.08.2006
(73) Patentinhaber: Activaero GmbH, 35285 Gemünden (DE)
(72) Erfinder: Müllinger, Bernhard, 80634 München (DE); Fischer, Axel, 34630 Moischeid (DE); Körber, Dorothee, 86157 Augsburg (DE); Wenker, Andreas, 86899 Landsberg am Lech (DE); Kolb, Tobias, 80687 München (DE); Roeder, Sascha, 80992 München (DE); Scheuch, Gerhard, 35288 Wohratal (DE)
(74) Vertreter: Vossius & Partner

(56) Entgegenhaltungen:
- US-A- 1 395 948
- US-A- 4 807 617
- US-A- 4 991 576
- US-A- 5 020 530
- US-A- 5 727 542
- US-A- 5 871 011
- US-A- 6 076 524
- US-A- 6 135 109
- US-A1- 2002 017 296
- US-B1- 6 631 721

## Beschreibung

Die vorliegende Erfindung betrifft ein Bauteil für eine Inhalationsvorrichtung, insbesondere für eine Inhalationsvorrichtung zur Inhalation von toxischen Wirkstoffen. Ferner betrifft die vorliegende Erfindung eine Inhalationsvorrichtung mit einem erfindungsgemäßen Bauteil.

US-A-5 871 011 betrifft eine Vorrichtung zum Liefern eines Anästhesiegases zu einem Patienten und wird als nächstliegender Stand der Technik für die vorliegende Erfindung angesehen. Diese Vorrichtung weist eine Maske auf, die Mund und Nase eines Patienten überdeckt, aber nicht am Gesicht anliegt. Zugeführtes Gas wird über einen Strömungskanal und über ein Mundstück in den Mund des Patienten geführt. Für die Exhalation ist ebenfalls ein Strömungskanal vorgesehen. Wenn der Patient ausatmet, strömt das Exhalat über ein Ventil in den Exhalationsströmungskanal. Für den Fall, dass der Patient nicht über das Mundstück ausatmet, sondern beispielsweise über die Nase in das Maskeninnere, strömt auch dieses Exhalat in den Strömungskanal für die Exhalation.

Die Verwendung des Inhalationswegs für die Applikation von Medikamenten gewinnt zunehmend an Bedeutung. Dabei werden außer dem Einsatz neuer lokal wirkender Medikamente für die Therapie von Lungenerkrankungen auch neue Therapiestrategien entwickelt, die die Lunge als Eingangsorgan für systemisch wirkende Substanzen verwenden.

Unter Umständen sind durch Inhalation toxische Wirkstoffe zu applizieren. Beispielsweise können bei einem Patienten mit Lungenkarzinom Zytostatika (z.B. Cisplatin) oder Zytokine verabreicht werden. Bei der Verabreichung von toxischen Wirkstoffen besteht immer die Notwendigkeit, die Belastung der Umwelt oder evtl. anwesender weiterer Personen mit diesen toxischen Wirkstoffen auszuschließen bzw. so gering wie möglich zu halten. Beispielsweise können während des Ausatemvorgangs Reste des toxischen Wirkstoffs wieder aus der Lunge des Patienten ausgeatmet werden. Herkömmliche Inhalationsgeräte tragen diesem Umstand nicht genügend Rechnung oder sind sehr unkomfortabel.

Der Erfindung liegt die Aufgabe zugrunde, die Inhalation toxischer Wirkstoffe unter Ausschaltung oder Verringerung möglicher Risiken oder einer Kontamination der Umgebung des behandelten Patienten zu ermöglichen. Diese Aufgabe wird mit einem Bauteil für eine Inhalationsvorrichtung sowie mit einer Inhalationsvorrichtung gemäß den Patentansprüchen gelöst.

Gemäß einem Hauptaspekt betrifft die vorliegende Erfindung ein Bauteil für eine Inhalationsvorrichtung. Das erfindungsgemäße Bauteil weist einen ersten Strömungskanal für die Inhalation auf, der sich zwischen einer ersten Lufteinlassöffnung und einer ersten Luftauslassöffnung erstreckt. Ferner ist ein zweiter Strömungskanal für die Exhalation vorgesehen, der sich zwischen einer zweiten Lufteinlassöffnung und einer zweiten Luftauslassöffnung erstreckt. Vorzugsweise fallen die erste Luftauslassöffnung und die zweite Lufteinlassöffnung zusammen, so dass ein und dieselbe Öffnung als Luftauslassöffnung für den ersten Strömungskanal und als Lufteinlassöffnung für den zweiten Strömungskanal dient. Alternativ dazu fallen die erste Lufteinlassöffnung und die erste Luftauslassöffnung, sowie die zweite Lufteinlassöffnung und die zweite Luftauslassöffnung zusammen, so dass lediglich eine erste und eine zweite Öffnung vorhanden sind, die die beiden Strömungskanäle darstellen.

Das erfindungsgemäße Bauteil weist ferner ein Filter auf, das in dem zweiten Strömungskanal angeordnet ist, bzw. dem zweiten Strömungskanal/der zweiten Öffnung zugeordnet ist, und beispielsweise in Verlängerung des zweiten Strömungskanals, d.h. stromabwärts, vorgesehen ist. Vorzugsweise wird der zweite Strömungskanal dann freigegeben und der erste Strömungskanal verschlossen, wenn die Inhalation durch den Patienten beendet wird, sei es durch Abbruch oder durch Zeitablauf am Ende der notwendigen Inhalationsdauer oder am Ende des Atemzuges.

Ferner weist das Bauteil eine Inhalationsmaske auf, in die der erste Strömungskanal endet und an der der zweite Strömungskanal beginnt. Die Inhalationsmaske weist ferner eine dritte Lufteinlassöffnung in Form eines Ventils für einen dritten Strömungskanal auf. Dieser dritte Strömungskanal gewährleistet einen kontinuierlichen Luftdurchfluss und verhindert, dass die Maske durch die Absaugung zu stark an das Gesicht gedrückt werden kann. Erfindungsgemäß erfolgt die Exhalation über den zweiten Strömungskanal.

Wenn das erfindungsgemäße Bauteil in einer Inhalationsvorrichtung verwendet wird, kann der Patient über den ersten Strömungskanal inhalieren. Dazu ist die erste Lufteinlassöffnung stromaufwärts mit einem Vernebler verbindbar, der den zu applizierenden Wirkstoff beispielsweise in Aerosolform in einen Druckluftstrom einbringt. Die Inhalation durch den Patienten erfolgt dann über ein Mundstück, das an der ersten Luftauslassöffnung abstromseitig des ersten Strömungskanals angeschlossen ist. Die Exhalation erfolgt in entgegengesetzter Richtung über das Mundstück über ein Exhalationsventil, jedoch dann nicht über den ersten Strömungskanal zurück in Richtung Vernebler, sondern über den zweiten Strömungskanal zu dem Filter, das in der Ausatemluft vorhandene Restbestände des toxischen Wirkstoffs herausfiltert. Es kann auch durch Entfernen vom Mundstück in die Maske exhaliert werden.

Vorzugsweise ist an das Filter stromabwärts eine Absaugeinrichtung angeschlossen. Dies hat den Vorteil, dass bei Beendigung der Inhalation nicht nur die ausgeatmete Luft des Patienten gefiltert wird, sondern auch aktiv der zweite Strömungskanal und das Mundstück abgesaugt und damit entleert werden, so dass auch nach Absetzen des Mundstücks keine toxischen Wirkstoffe aus der Mundstücköffnung in die Umgebung gelangen können. Vorzugsweise ist in diesem Absaugkanal ein Exhalationspuffer vorhanden, der im Falle zu niedriger Absaugraten die ausgeatmete Luft zwischenspeichert. Der Exhalationspuffer wird dann beispielsweise während des nächsten Inhalationsschrittes weiter entleert. Der Absaugfluss beträgt vorzugsweise zwischen 15 und 80 1/min.

Vorzugsweise bilden der erste Strömungskanal und der zweite Strömungskanal ein 3-Wege-Ventil. Die drei Öffnungen entsprechen (i) dem ersten Lufteinlass, (ii) dem ersten Luftauslass/zweiten Lufteinlass und (iii) dem zweiten Luftauslass. Alternativ dazu sind zwei getrennte Strömungskanäle vorgesehen, wobei beispielsweise an der ersten Luftauslassöffnung ein Einwegventil vorgesehen ist, das lediglich eine Inhalation, also einen Luftstrom zum Patienten, zulässt, und an der zweiten Lufteinlassöffnung ebenfalls ein Einwegventil vorgesehen ist, das lediglich eine Exhalation zulässt. Dadurch ist sichergestellt, dass die Exhalation nur über den zweiten Strömungskanal erfolgt.

In einer bevorzugten Ausführungsform ist ferner abstromseitig von der ersten Luftauslassöffnung bzw. der zweiten Lufteinlassöffnung ein Schutzelement vorgesehen, das vorzugsweise selbst-expandierend ist, beispielsweise ein Faltenbalg. Dieses Schutzelement umgibt die erste Luftauslassöffnung bzw. die zweite Lufteinlassöffnung und ein gegebenenfalls vorhandenes Mundstück. Dabei ist das Schutzelement dichtend beispielsweise mit dem Ende des Mundstücks verbunden, das der Mundstückspitze gegenüber liegt. Das Schutzelement ist im Ruhezustand expandiert, ist aber bei Verwendung der Inhalationsvorrichtung komprimierbar. Im expandierten Zustand erstreckt sich das Schutzelement über die Spitze des Mundstücks hinaus, wie ein Auffangbehältnis. Wenn somit der Benutzer die Inhalationsvorrichtung vom Mund entfernt, expandiert das Schutzelement automatisch, um dadurch seine Schutzfunktion zu entfalten: die kontaminierte Luft bleibt im vorstehenden Schutzelement gefangen und kann nicht an die Umgebung entweichen, sondern wird abgesaugt und gefiltert. Um dem Benutzer das Ausatmen zu ermöglichen bzw. zu erleichtern, bevor der Inhalationsvorgang beginnt, oder in einer Pause, weist das Schutzelement vorzugsweise ein Ausatemventil auf. Somit kann der Benutzer in das Innere des Schutzelementes ausatmen und die Luft entweicht dann über das Ausatemventil. Besonders bevorzugt erstreckt sich das Schutzelement über das gesamte erfindungsgemäße Bauteil (vergleichbar mit einem Ballon), so dass alle eventuellen Undichtigkeiten abgedeckt werden und keine kontaminierte Luft in die Umgebung entweichen kann, sondern vom Schutzelement abgehalten wird.

Der Überstand des Schutzelementes beträgt vorzugsweise zwischen 5 und 100 mm. Der Absaugfluss beträgt hingegen vorzugsweise zwischen 15 und 80 1/min. Je größer der Überstand, desto geringer kann der Absaugfluss sein, bei dem noch kein Austritt des Aerosols erfolgt.

Die Inhalationsmaske des Bauteils gemäß der vorliegenden Erfindung ist vorzugsweise derart gestaltet und dimensioniert, dass sie den Mund und die Nase des Benutzers überdecken kann, so dass jegliche über den Mund oder die Nase ausgeatmete Luft in das Maskeninnere gelangt. Beispielsweise ist diese eine aus der Beatmung bekannte CPAP (Continuous Positive Airway Pressure) Maske. Gemäß einer ersten Alternative weist die Inhalationsmaske eine Öffnung auf, an der beispielsweise eine Schlauchverbindung zwischen den Strömungskanälen und der Inhalationsmaske endet (die Strömungskanäle können auch direkt in das Maskeninnere münden). Die Inhalationsmaske schließt hier vorzugsweise bündig mit der Schlauchverbindung ab, so dass die Inhalationsmaske wie ein trichterförmiges Element die Schlauchverbindung verlängert. In dieser Alternative ist ein Mundstück, das der Patient in den Mund nimmt, nicht zwingend vorgesehen. Alternativ dazu ist ein Mundstück vorhanden und durchdringt die Inhalationsmaske und ragt in den Maskeninnenraum, der zwischen der Inhalationsmaske und dem Gesicht des Benutzers gebildet wird, hinein. Vorzugsweise ragt das Mundstück derart weit in die Inhalationsmaske hinein, dass der Benutzer das Mundstückende bzw. die Mundstückspitze zur Inhalation/Exhalation leicht in den Mund nehmen kann. In dieser Ausführungsform sieht eine bevorzugte Ausgestaltung vor, dass das Mundstück relativ zur Inhalationsmaske verschiebbar ist. Dabei ist das Mundstück zwischen einer ersten Position (der Inhalationsstellung), in der der Benutzer das Mundstück in den Mund nehmen kann, und einer zweiten Position (der Exhalationsstellung), in der das Mundstück in den Maskeninnenraum zurückgezogen ist, verschiebbar. Dies hat den Vorteil, dass der Benutzer das Mundstück zur Inhalation in den Mund nehmen kann, zur Exhalation dieses aber freigibt und zurückzieht, so dass die Exhalation nicht in Richtung des Verneblers erfolgt, sondern in den Maskeninnenraum. Außerdem kann der Benutzer so zwischendurch einatmen ohne zu inhalieren ohne die Maske abzunehmen.

Vorzugsweise ist hierzu ein flexibles Element als Bindeglied zwischen dem Mundstück und der Maske vorgesehen. Mehr bevorzugt ist das flexible Element selbst-expandierend, so dass das Mundstück, wenn es vom Patienten freigegeben wurde, automatisch durch die Expansion des flexiblen Elements zurückgezogen wird.

Gemäß einer weiter bevorzugten Ausführungsform ist die Inhalationsmaske des Bauteils im Umfangsbereich derart ausgebildet, dass im Falle eines Unterdrucks im Maskeninnenraum ein Druckausgleich über den Umfangsbereich der Inhalationsmaske möglich ist. Dies bedeutet, dass der Umfangsbereich der Inhalationsmaske so ausgestaltet ist, dass er nicht absolut dichtend an der Haut des Patienten anliegt, sondern den Zufluss von Luft in den Maskeninnenraum im Falle eines Unterdrucks zulässt. Dies ist besonders vorteilhaft, wenn eine Absaugeinrichtung die im Inhalationsmaskeninnenraum vorhandene Luft über eine entsprechende Öffnung absaugt, denn dann kann die Absaugeinrichtung, beispielsweise als Sicherheitsvorkehrung, kontinuierlich betrieben werden, und im Falle eines Unterdrucks strömt Umgebungsluft zum Druckausgleich in den Maskeninnenraum. Mit dem toxischen Wirkstoff kontaminierte Ausatemluft kann jedoch nicht über den Umfangsbereich der Inhalationsmaske entweichen, da die Absaugeinrichtung für deren Absaugung sorgt.

Alternativ zu der Möglichkeit, Luftzufluss über den Maskenrand zuzulassen, weist die Maske Einwegventile auf, die für den nötigen Druckausgleich sorgen. Diese sind vorzugsweise im vorderen, also vom Mund des Benutzers entfernten Bereich der Maske vorgesehen.

Die Inhalationsmaske ist vorzugsweise ballonförmig ausgebildet. Vorzugsweise durchdringt das Mundstück die derart ballonförmig ausgebildete Inhalationsmaske in einem ersten Bereich, der dem Gesicht des Benutzers abgewandt ist, und erstreckt sich in den Maskeninnenraum. Ein diesem Bereich gegenüberliegender zweiter Bereich, der bei der Inhalation dem Gesicht des Benutzers zugewandt ist, ist vor der Inhalation verschlossen und wird erst zu Beginn der Inhalation dadurch von der Mundstückspitze des Mundstücks durchdrungen, dass der Benutzer diesen Maskenbereich an sein Gesicht anlegt und das im Inneren der Inhalationsmaske befindliche Mundstückende zum Mund verschiebt.

Das erfindungsgemäße Bauteil ist vorzugsweise Bestandteil des Aerosolgenerators der Inhalationsvorrichtung.

Ein weiterer erfindungsgemäßer Aspekt betrifft eine Inhalationsvorrichtung mit einem Bauteil nach dem Hauptaspekt der Erfindung.

Gemäß einer bevorzugten Ausführungsform des weiteren Aspekts der vorliegenden Erfindung wird eine Inhalationsvorrichtung mit einem erfindungsgemäßen Bauteil gemäß dem Hauptaspekt sowie mit einem Vernebler, der mit der ersten Lufteinlassöffnung verbunden ist und einem Mundstück, das mit der ersten Luftauslassöffnung und der zweiten Lufteinlassöffnung verbunden ist, bereitgestellt. Vorzugsweise weist die Inhalationsvorrichtung eine Absaugeinrichtung auf, die mit der zweiten Luftauslassöffnung des Bauteils derart verbunden ist, dass ausgeatmete Luft über das Filter des Bauteils abgesaugt wird.

Alternativ dazu weisen die Verhinderungsmittel eine Inhalationsmaske mit einer ersten Öffnung für die Inhalation und einer Absaugöffnung auf, ferner mit einem mit der ersten Öffnung verbundenen Vernebler und einer mit der Absaugöffnung verbundenen Absaugeinrichtung.

Vorzugsweise schaltet die erfindungsgemäße Inhalationsvorrichtung die Verneblung des Wirkstoffs nur dann ein, wenn der Patient oder der Benutzer am Mundstück einen leichten Unterdruck erzeugt und so der Wille des Benutzers zur Inhalation erkennbar ist. Ebenso schaltet die Verneblung vorzugsweise unverzüglich ab, wenn der Unterdruck am Mundstück nachlässt.

Die Absaugung der ausgeatmeten Luft des Benutzers wird vorzugsweise erst dann zugeschaltet, wenn überhaupt vom Vernebler Aerosol erzeugt wird, da lediglich dann die Gefahr besteht, toxische Wirkstoffe an die Umgebung auszuatmen. Dies bedeutet, dass die Absaugung auch erst dann beginnt, wenn der Benutzer/Patient mit der Inhalation beginnt, da dies die Verneblung startet. Sofern der Patient während der Inhalation stoppt, wird die Absaugung fortgeführt, und die Absaugung vorzugsweise sogar erhöht, um umgehend die Strömungskanäle zu entleeren. Bei einer regulären Beendung der Inhalation stoppt zwar die Verneblung des Wirkstoffs, die Absaugung wird jedoch fortgeführt, da nun erst die Exhalation beginnt. Auch nach erfolgter Exhalation durch den Benutzer wird die Absaugung noch für einen gewissen Zeitbereich durchgeführt, um eventuelle Restbestände kontaminierter Ausatemluft abzusaugen.

Die Erfindung wird nun unter Bezugnahme auf die beigefügten Zeichnungen näher erläutert. Es zeigen:
- Fig. 1a: ein Bauteil gemäß einer ersten erfindungsgemäßen Ausführungsform während der Inhalation;
- Fig. 1b: das Bauteil von Fig. 1a während der Exhalation;
- Fig. 2: eine detaillierte Darstellung des Bauteils von Fig. 1 zusammen mit optionalen Merkmalen;
- Fig. 3: das Bauteil von Fig. 2 während der Inhalationspause;
- Fig. 4: eine perspektivische Ansicht eines praktischen Beispiels des Bauteils von Fig. 2;
- Fig. 5: den prinzipiellen Aufbau einer Inhalationsvorrichtung für die Inhalation toxischer Medikamente;
- Fig. 6a: ein Bauteil das nicht zur Erfindung gehört;
- Fig. 6b: die Verwendung des Bauteils von Fig. 6a durch einen Patienten;
- Fig. 7: ein Bauteil gemäß einer zweiten erfindungsgemäßen Ausführungsform;
- Fig. 8: ein Bauteil gemäß einer dritten erfindungsgemäßen Ausführungsform;
- Fig. 9: ein Bauteil einer vierten erfindungsgemäßen Ausführungsform;
- Fig. 10: die Verwendung einer bevorzugten Inhalationsmaske während des Inhalationsvorgangs;
- Fig. 11: die Verwendung einer bevorzugten Inhalationsmaske während der Exhalation;
- Fig. 12: eine schematische Darstellung einer Inhalationsmaske;
- Fig. 13: eine weiter alternative Ausführungsform der Inhalationsmaske; und
- Fig. 14: eine weiter alternative Ausführungsform der Inhalationsmaske;

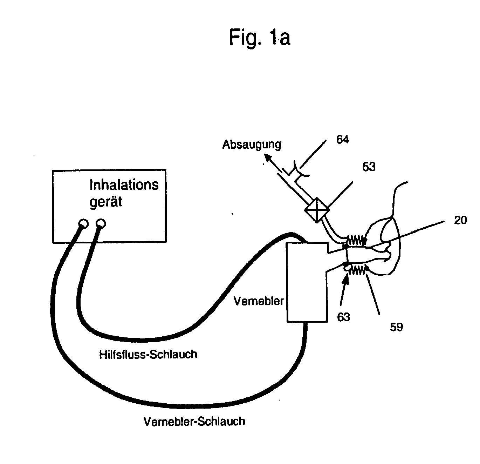

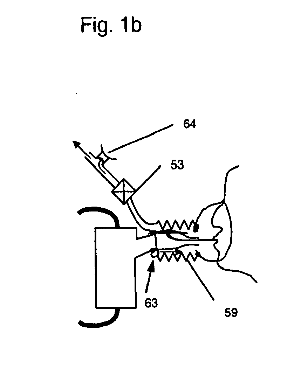

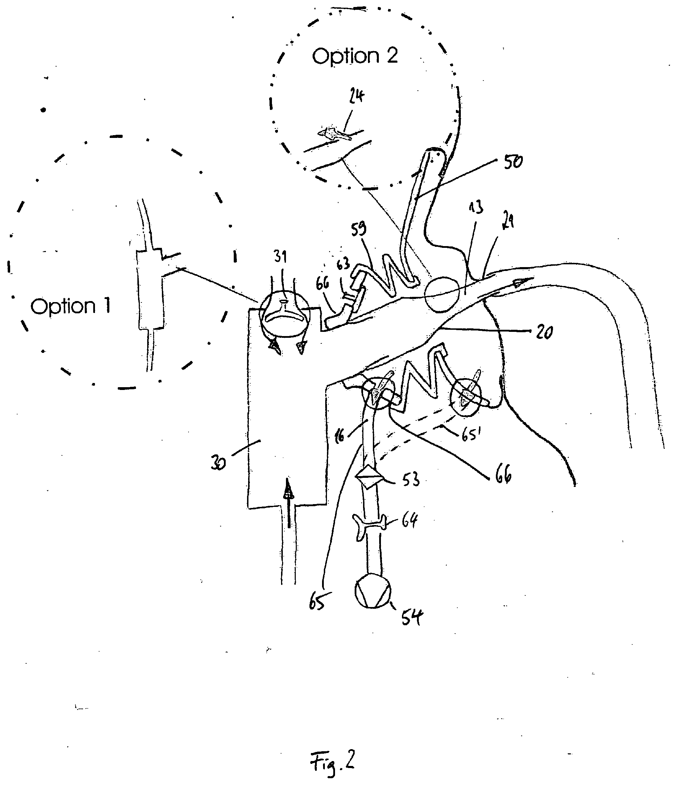

Fig. 1a und 1b zeigen eine erste Ausführungsform eines erfindungsgemäßen Bauteils. An einen Vernebler 30 ist ein Mundstück 20 angeschlossen, das der Patient zur Inhalation in den Mund nehmen kann. Dies stellt einen ersten Strömungskanal 13 für die Inhalation dar. Das Mundstück 20 ist von einem flexiblen Schutzelement 59 umgeben, das vorzugsweise selbst-expandierend ist, beispielsweise ein Faltenbalg. Dieses Schutzelement 59 ist im Ruhezustand expandiert, wie beispielsweise in Fig. 1 bund 3 gezeigt, ist aber bei Verwendung der Inhalationsvorrichtung komprimierbar, wie in Fig. 1a und 2 gezeigt. Im expandierten Zustand erstreckt sich das Schutzelement 59 vorzugsweise über die Spitze 21 des Mundstücks 20 hinaus. Wenn somit der Benutzer die Inhalationsvorrichtung bzw. deren Bauteil-Mundstück 20 vom Mund entfernt, expandiert das Schutzelement 59 automatisch, um dadurch seine Schutzfunktion zu entfalten: kontaminierte Luft bleibt im Schutzelement 59 gefangen und kann nicht an die Umgebung entweichen, sondern wird abgesaugt und gefiltert, wie noch beschrieben wird.

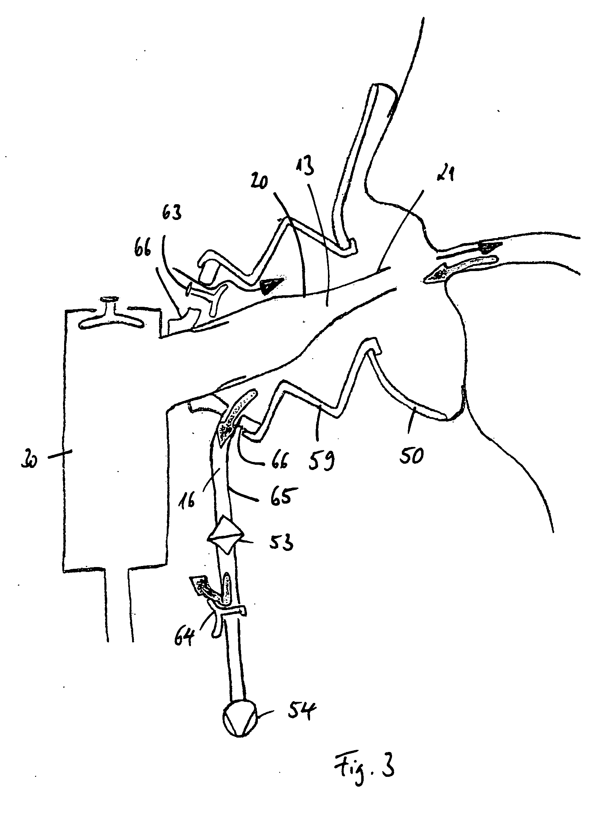

Das in den Figuren 1a, 1b und 2 bis 4 gezeigte Bauteil weist ferner einen Exhalationsweg 65 auf. Dieser stellt einen zweiten Strömungskanal 16 für die Exhalation dar. Dieser Exhalationsweg 65 setzt vorzugsweise an der Verbindungsstelle 66 von Mundstück 20 und flexiblem Element 59 an, wie in Fig. 2 und 3 gut zu sehen ist. Um dem Benutzer das Ausatmen zu ermöglichen bzw. zu erleichtern, bevor der Inhalationsvorgang beginnt, oder in einer Inhalationspause (Fig. 3), weist der Exhalationsweg 65 vorzugsweise ein Exhalations- bzw. Exspirationsventil 64 auf. An den Exhalationsweg 65 ist vorzugsweise eine Absaugeinrichtung 54 angeschlossen, die die vom Patienten ausgeatmete kontaminierte Luft über ein Exhalationsfilter 53 absaugt. Das Exspirationsventil 64 bietet den Vorteil, dass der Patient mit stärkerem Fluss ausatmen kann als die Absaugeinrichtung 54 absaugt. Außerdem kann er bei einem Ausfall der Absaugeinrichtung 54 durch Weiteratmen die kontaminierte Luft aus dem System in das Filter 53 befördern. Dies bedeutet, die Exhalation erfolgt über das Filter 53 und dann entweder über die Absaugung 54 und/oder das Exspirationsventil 64.

An der dem Patienten zugewandten Ende des flexiblen Elements 59 ist bei der Ausführungsform der Figuren 1 a bis 4 eine Maske 50 angebracht, die Mund und Nase des Patienten überdeckt, so dass kontaminierte Luft lediglich in das Innere des flexiblen Elements 59 und damit zum Exspirationsfilter 53 hin strömen bzw. entweichen kann. Wie in Fig. 2 gezeigt ist, kann der Exhalationsweg 65' alternativ auch an der Maske 50 beginnen, also direkt zum Maskeninneren hin geöffnet sein und nicht zum Faltenbalg 59.

Wie beschrieben beginnt der Exhalationsweg 65 bevorzugt an der Verbindungsstelle 66 von Mundstück 20 und flexiblen Element 59. Bei der Ausführungsform von Fig. 2 und 3 ist in diesem Bereich ein Inspirationsventil 63 angeordnet. Dieses erlaubt einen Druckausgleich während der Exhalation sowie eine Einatmung vor Beginn des Inhalationsvorganges oder in Inhalationspausen.

Optional weist das Mundstück 20 selbst ein Ventil 24 auf. Dies ist mit dem Vorteil verbunden, dass der Patient das Mundstück 20 beim Ausatmen nicht notwendigerweise aus dem Mund nehmen muss. Eine Exhalation gegen den Widerstand des Verneblers 30 bewirkt eine Öffnung des Mundstückventils 24, so dass auch dann die kontaminierte Luft in das Innere der Maske 50 und des Faltenbalgs 59 gelangt und abgesaugt werden kann.

Der an das Mundstück 20 angeschlossene Vernebler 30 kann entweder ein Vernebler sein, dem das Aerosol von einer Inhalationspumpe zugeführt wird, Zuluft jedoch über ein entsprechendes Ventil 31 erhält, oder aber ein Vernebler mit kontrollierter Zuluft, wie etwa der Vernebler des Systems AKITA der Anmelderin. Letzteres ist als Option 1 in Fig. 2 gezeigt.

Die jeweiligen Luftströme sind in den Figuren 2 und 3 mit dünnen Pfeilen (Aerosol, Inspiration) bzw. dicken Pfeilen (Exhalat, Exspiration) verdeutlicht.

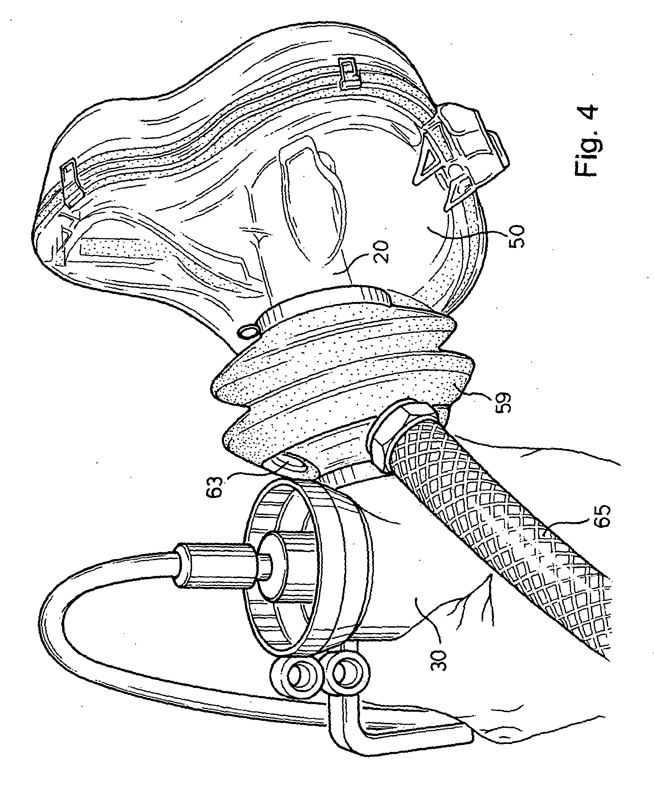

Fig. 4 zeigt eine praktische Realisierung des erfindungsgemäßen Bauteils gemäß Figuren 2 und 3. Fig. 4 zeigt deutlich den an den Vernebler angeschlossenen Faltenbalg 59 samt Maske 50 und Mundstück 20, sowie den Exhalationsschlauch 65, der zur Absaugung führt.

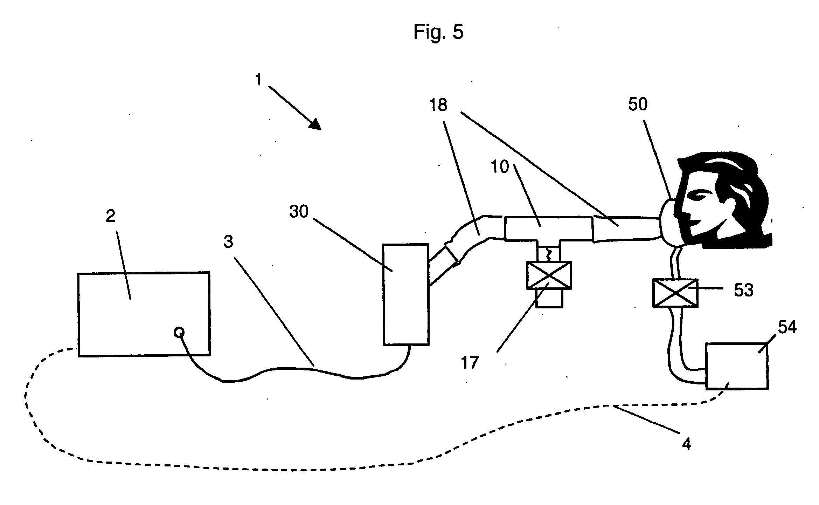

Fig. 5 zeigt den prinzipiellen Aufbau einer erfindungsgemäßen Inhalationsvorrichtung 1 für die Inhalation toxischer Medikamente. Diese Inhalationsvorrichtung weist ein Inhalationsgerät 2 auf, das die Steuerelektronik, Eingabetasten, Display etc. enthält und über ein entsprechendes Verbindungskabel 3 für Luft, Daten und Strom mit einem Vernebler 30 verbunden ist. In dem Vernebler 30 wird der zu applizierende Wirkstoff zerstäubt und in einen Druckluftstrom eingebracht, der über einen entsprechenden Schlauch 18, das erfindungsgemäße Bauteil 10 und einen weiteren Schlauch 18 (oder ein Mundstück (nicht gezeigt)) mit einer Maske 50 verbunden ist. Diese Maske 50 trägt der Patient, Mund und Nase überdeckend, an seinem Gesicht. Das erfindungsgemäße Bauteil 10 weist ein Filter 17 auf, das bei der Inhalation toxischer Wirkstoffe die in der Ausatemluft vorhandenen Restbestände dieser Wirkstoffe aus der Ausatemluft herausfiltert und somit die Ausatemluft reinigt. Entsprechend ist gemäß Fig. 5 vorzugsweise ein weiteres Filter 53 vorgesehen, das über einen entsprechenden Schlauch direkt mit dem Innenraum der Inhalationsmaske 50 verbunden ist. Eine Absaugeinrichtung 54, die ebenfalls von dem Inhalationsgerät 2 über eine Verbindungsleitung 4 angesteuert wird, saugt die ausgeatmete Luft über das Filter 53 aus der Inhalationsmaske 50 heraus.

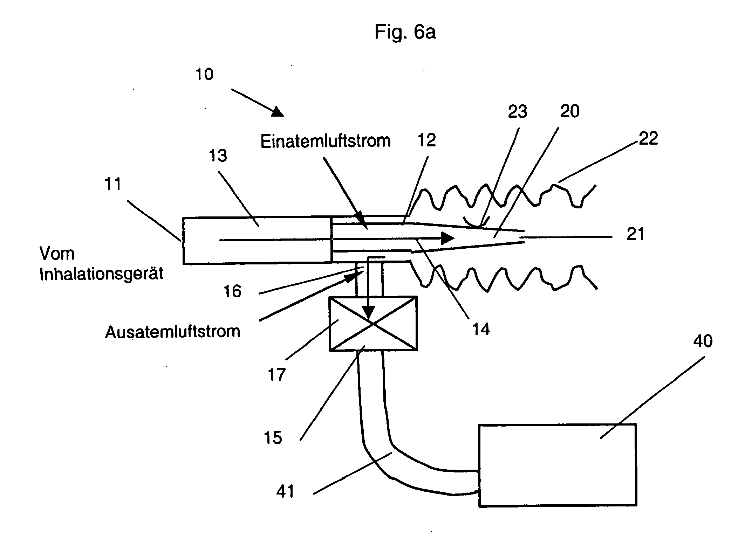

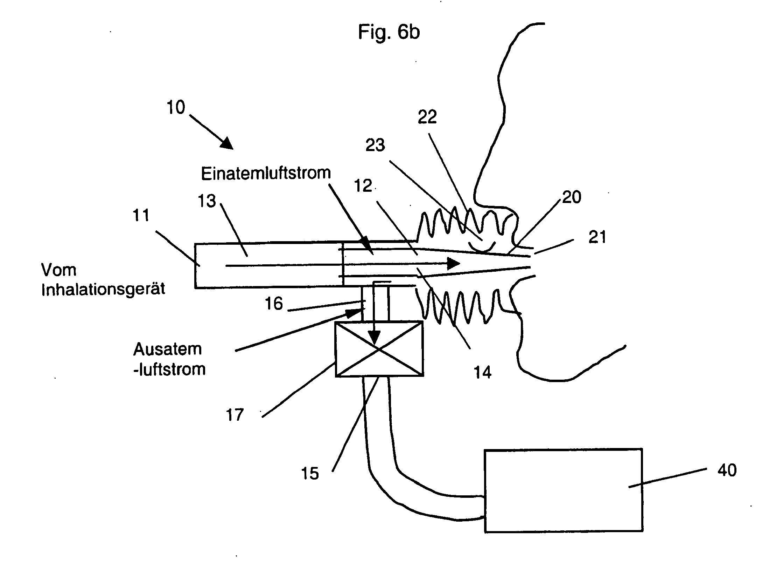

Gemäß Fig. 6a weist das Bauteil 10 einen ersten Strömungskanal 13 zwischen einer ersten Lufteinlassöffnung 11 und einer ersten Luftauslassöffnung 12 auf. Über diesen ersten Strömungskanal 13 erfolgt die Inhalation vom Vernebler und über das Mundstück 20. Der Patient nimmt dazu das Mundstückende bzw. die Mundstückspitze 21 in den Mund, wie in Fig. 6b gezeigt. Für die Exhalation ist ein zweiter Strömungskanal 16 vorgesehen, der sich zwischen einer zweiten Lufteinlassöffnung 14 und einer zweiten Luftauslassöffnung 15 erstreckt. In der in den Fig. 6a und 6b gezeigten Form fallen die erste Luftauslassöffnung 12 und die zweite Lufteinlassöffnung 14 zusammen. In dieser Form ist das Bauteil 10 als 3-Wege-Ventil ausgebildet, das eine Inhalation über den ersten Strömungskanal 13, eine Exhalation jedoch lediglich über den zweiten Strömungskanal 16 zulässt. Im zweiten Strömungskanal 16 oder diesem zugeordnet ist ein Filter 17 vorgesehen, das die ausgeatmete Luft reinigt und dekontaminiert. Gemäß der Form von Fig. 6a/6b ist an den zweiten Strömungskanal 16 über einen Schlauch 41 eine Absaugeinrichtung 40 angeschlossen, die die ausgeatmete Luft absaugt.

In der Form von Fig. 6a ist ferner ein Schutzelement 22 vorgesehen, das vorzugsweise selbst-expandierend ist, beispielsweise ein Faltenbalg. Dieses Schutzelement 22 ist im Ruhezustand expandiert, wie in Fig. 6a gezeigt, ist aber bei Verwendung der Inhalationsvorrichtung komprimierbar, wie in Fig. 6b gezeigt, so dass der Patient das Mundstück in den Mund nehmen kann. Im expandierten Zustand erstreckt sich das Schutzelement 22 über die Spitze 21 des Mundstücks 20 hinaus. Wenn somit der Benutzer das Bauteil der Inhalationsvorrichtung vom Mund entfernt, expandiert das Schutzelement 22 automatisch, um dadurch seine Schutzfunktion zu entfalten: kontaminierte Luft bleibt im Schutzelement 22 gefangen und kann nicht an die Umgebung entweichen, sondern wird abgesaugt und gefiltert. Um dem Benutzer das Ausatmen zu ermöglichen bzw. zu erleichtern, bevor der Inhalationsvorgang beginnt, oder in einer Pause, weist das Schutzelement 22 vorzugsweise ein Ausatemventil 23 auf. So kann der Patient ausatmen, auch wenn der Faltenbalg an seinem Gesicht anliegt (Fig. 6b).

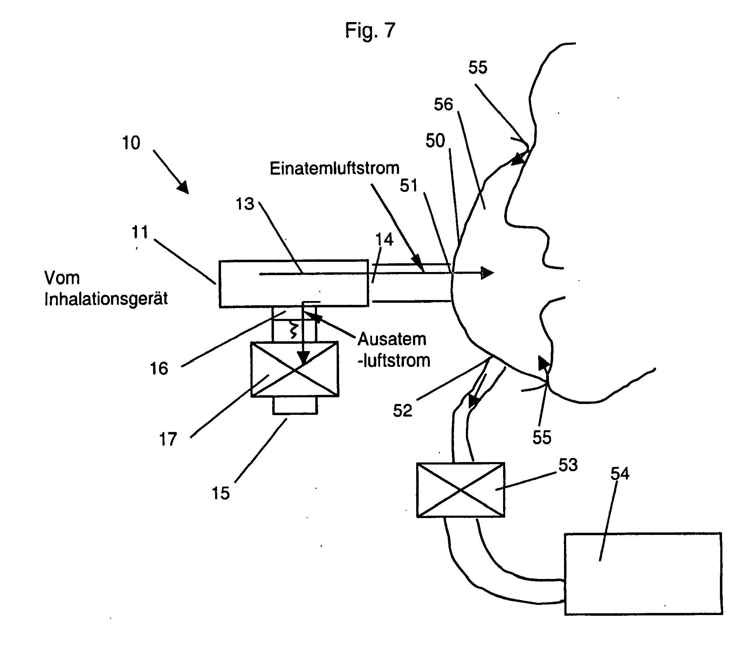

Eine alternative bevorzugte Ausführungsform des erfindungsgemäßen Bauteils ist in Fig. 7 gezeigt. Hier ist im Unterschied zu dem Bauteil von Fig. 2 kein Mundstück 20 vorgesehen, das der Patient in den Mund nimmt. Vielmehr endet der Inhalationsluftstrom in einer Inhalationsmaske 50, die derart dimensioniert ist, dass sie Mund und Nase des Patienten überdeckt. Ein "Mundstück" bzw. eine Schlauchverbindung erstreckt sich von der ersten Luftauslassöffnung 12 bis zu einer Öffnung 51 in der Inhalationsmaske 50. Ferner ist in der Inhalationsmaske 50 eine zweite Öffnung 52 vorgesehen, die den Inhalationsmaskeninnenraum 56 über ein Filter 53 mit einer Absaugeinrichtung 54 verbindet. Über diesen Kanal kann ausgeatmete Luft abgesaugt werden. Überdies wird ausgeatmete Luft über das erfindungsgemäße Bauteil 10 und insbesondere über den zweiten Strömungskanal 16 und das Filter 17 abgeführt.

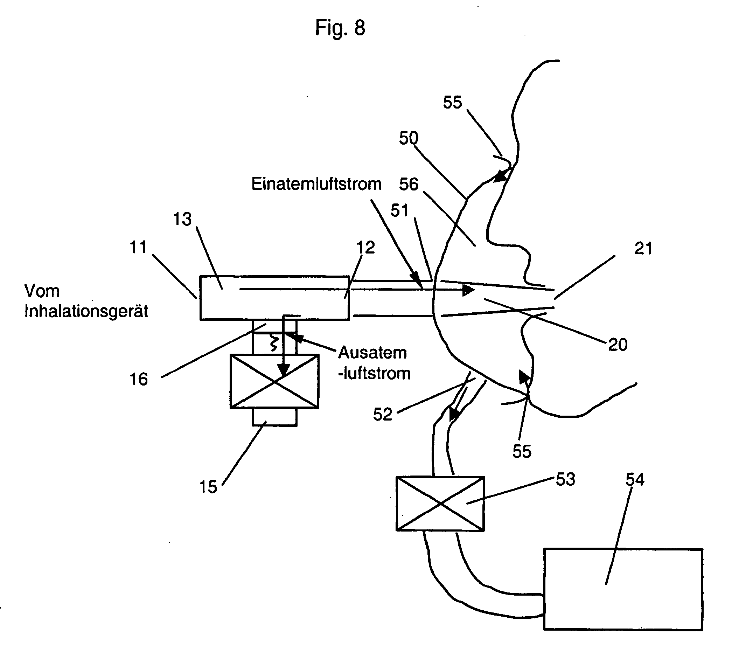

Fig. 8 zeigt eine zu Fig. 7 ähnliche Ausführungsform. In der Ausführungsform von Fig. 8 ist jedoch noch zusätzlich ein Mundstück 20 vorgesehen. Das Mundstück 20 erleichtert dem Patienten die Inhalation, da er dieses an dessen Ende 21 in den Mund nehmen kann und bequem lediglich über diesen Strömungskanal inhaliert. Die Ausatmung erfolgt in dieser Ausführungsform beispielsweise über die Nase, so dass der Patient das Mundstück im Mund belassen kann. Derart ausgeatmete Luft wird wiederum über das Filter 53 von der Absaugeinrichtung 54 direkt aus dem Maskeninneren 56 abgesaugt. Um jedoch im Falle eines Unterdrucks im Maskeninneren 56 zu verhindern, dass die Maske 50 auf das Gesicht des Patienten gezogen wird, ist die Inhalationsmaske 50 im Umfangsbereich 55 derart ausgebildet, dass dort ein Lufteinlass möglich ist. Wie oben erläutert sind dazu Einwegventile vorgesehen, die für einen notwendigen Druckausgleich sorgen. Sobald also beispielsweise über die Absaugeinrichtung 54 ein Unterdruck im Maskeninneren 56 entsteht, kann Umgebungsluft über den Umfangs- bzw. Randbereich 55 oder ein Einwegventil (nicht gezeigt) nachfließen. Entsprechendes gilt für die in Fig. 7 gezeigte bevorzugte Ausführungsform.

Wenn in der Ausführungsform von Fig. 8 nicht nur über die Nase sondern auch oder ausschließlich über den Mund exhaliert wird, wird die exhalierte Luft über den zweiten Strömungskanal 16 und das Filter 17 abgeführt und gereinigt.

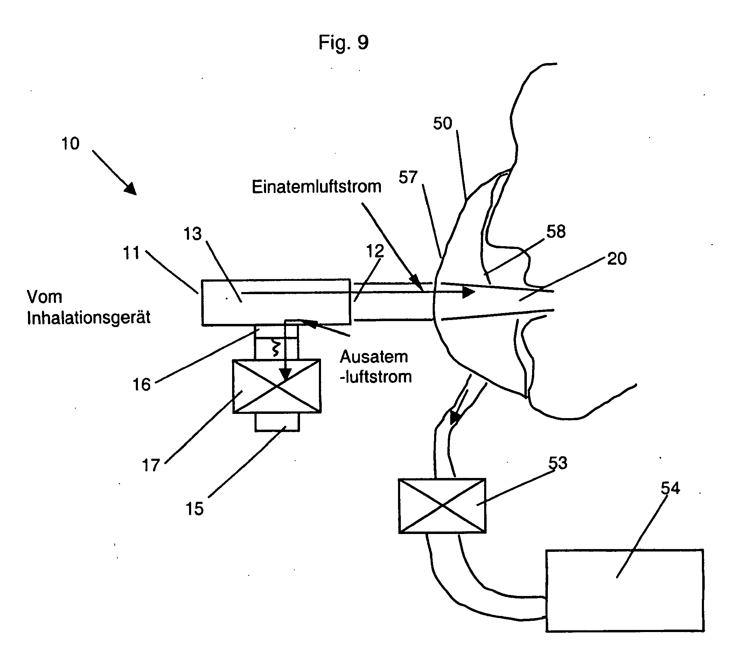

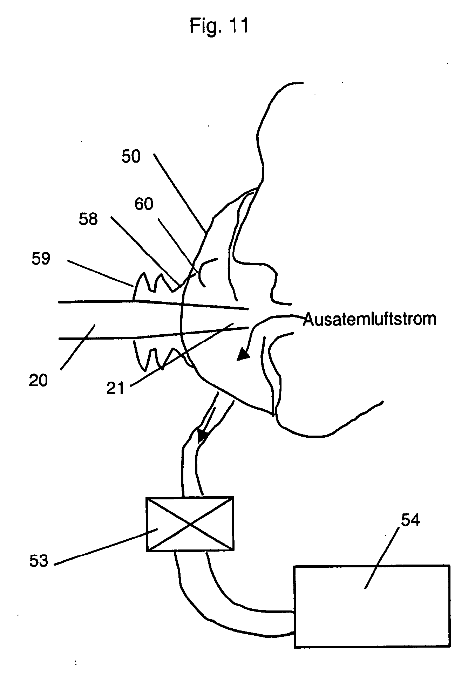

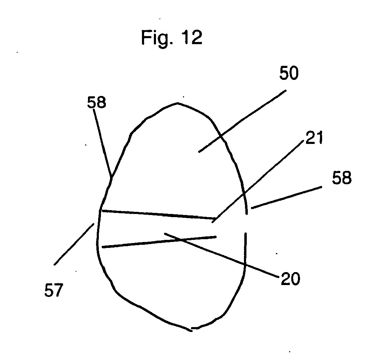

Fig. 9 zeigt eine weitere Ausführungsform des erfindungsgemäßen Bauteils 10. Gemäß dieser Ausführungsform wird eine besonders bevorzugte Inhalationsmaske 50 verwendet, die in Fig. 12 näher dargestellt ist. Diese Inhalationsmaske 50 ist ballonförmig ausgebildet und wird in einem ersten Bereich 57 vom Mundstück 20 durchdrungen. Dieser erste Bereich 57 ist der Bereich der Inhalationsmaske 50, der während der Inhalation vom Gesicht des Patienten abgewandt ist. Der gegenüberliegende Bereich 58 ist hingegen während der Inhalation dem Gesicht des Patienten zugewandt. Vor der Inhalation ragt der Bereich 58 wie in Fig. 11 gezeigt über die Mundstückspitze 21 hinaus. Auch dies stellt eine Schutzfunktion wie oben im Zusammenhang mit Fig. 6 erläutert bereit. Eventuell ausströmende kontaminierte Luft wird in der Maske 50 gefangen und abgesaugt. Sobald jedoch der Patient die Inhalationsmaske anlegt und an das Gesicht drückt, überragt die Spitze bzw. das Ende 21 des Mundstücks 20 den Bereich 58 der Inhalationsmaske, so dass der Patient das Mundstück in den Mund nehmen kann. Der Bereich 58 wird dabei lediglich auf das Mundstück 20 geschoben. Dies ist in Fig. 9 gezeigt.

Fig. 9 zeigt die erfindungsgemäße Inhalationsmaske bereits im aufgesetzten Zustand. Ansonsten ist diese bevorzugte Ausführungsform identisch zu der von Fig. 8, so dass auf die Erläuterungen zu Fig. 8 verwiesen wird.

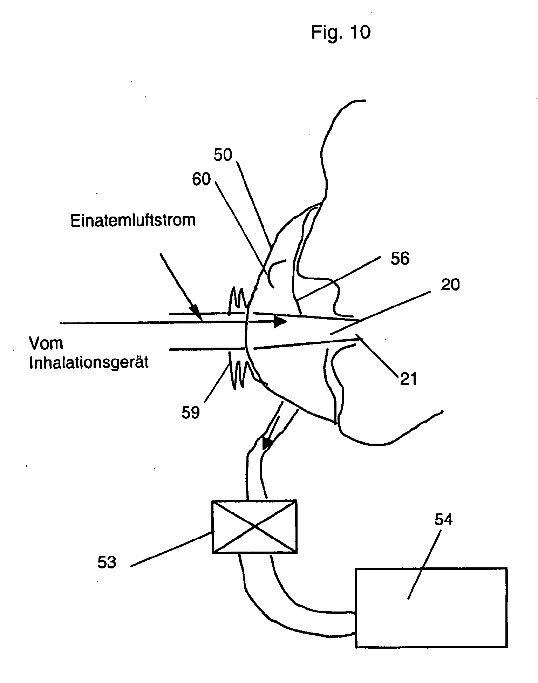

Die Figuren 10 und 11 zeigen eine weitere Variante der Ausbildung von Inhalationsmaske 50 und Mundstück 20. Gemäß dieser bevorzugten Ausführungsform ist das Mundstück 20 relativ zur Inhalationsmaske 50 flexibel gelagert bzw. verschiebbar angeordnet. Fig. 10 zeigt das Mundstück relativ zur Inhalationsmaske in einer Position, in der der Patient inhaliert. Zur Erleichterung der Ausatmung ist jedoch das Mundstück zurückziehbar, d.h. vom Patienten entfernbar, wie in Fig. 11 gezeigt. Der Patient lässt dazu das Mundstückende 21 los und es wird ausreichend weit in den Maskeninnenraum 56 zurückgezogen, so dass der Patient bequem ausatmen kann. Die ausgeatmete Luft wird dann beispielsweise über die Absaugeinrichtung 54 abgesaugt. Zur flexiblen bzw. verschiebbaren Lagerung des Mundstücks 20 ist wie in den Figuren 10 und 11 gezeigt beispielsweise ein Faltenbalg 59 oder ähnliches flexibles Element vorgesehen. Wenn der Patient wie in Fig. 10 gezeigt das Mundstück 20 in den Mund schiebt wird dieses flexible Element 59 zusammengedrückt. Im Falle eines selbst-expandierbaren flexiblen Elementes 59 zieht sich das Mundstück 20 automatisch zurück, wenn es der Patient freigibt.

Die Ausführungsformen von Fig. 10 und 11 zeigen auch das zum Druckausgleich vorhandene Einwegeventil 60 der Maske 50.

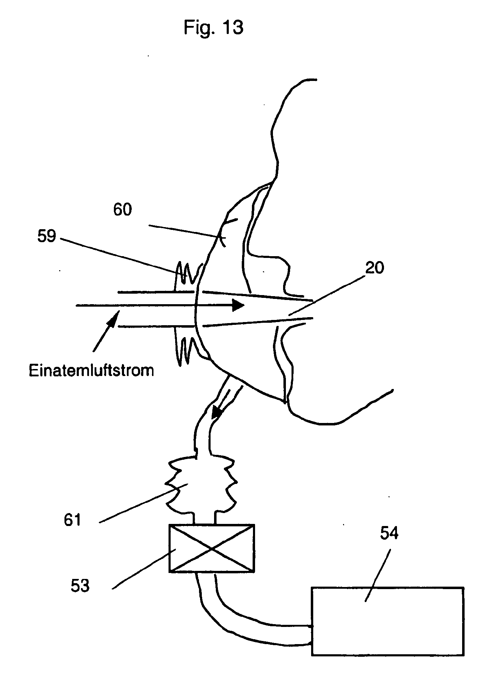

Eine weitere Alternative ist in Fig. 13 gezeigt. Diese entspricht im Wesentlichen der Ausführungsform von Fig. 10. In Fig. 13 ist jedoch zusätzlich ein Pufferelement 61 gezeigt, das als Ausatempuffer bzw. Exhalationsbeutel dient. Im Falle einer zu geringen Absaugrate ist so sichergestellt, dass der Patient trotzdem normal ausatmen kann. Nicht abgesaugte exhalierte Luft wird dann im Ausatempuffer gepuffert und verzögert abgesaugt, während der Patient wieder inhaliert. Ein derartiger Puffer kann in allen Ausführungsformen vorgesehen sein.

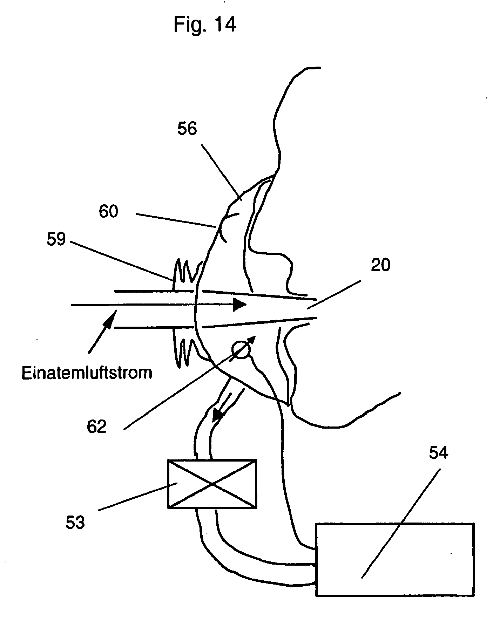

In der Ausführungsform von Fig. 14 ist weiterhin ein Drucksensor 62 vorgesehen. Dieser Drucksensor misst den Druck im Maskeninneren 56 und liefert das Ergebnis an die Absaugeinrichtung 54. Diese ist dann in der Lage, eine druckabhängige bzw. druckgeregelte Absaugrate einzustellen.

## Patentansprüche

1. Bauteil für eine Inhalationsvorrichtung (1) mit:
einer ersten Lufteinlassöffnung (11) und einer ersten Luftauslassöffnung (12), die über einen ersten Strömungskanal (13) für die Inhalation verbunden sind;
einer zweiten Lufteinlassöffnung (14) und einer zweiten Luftauslassöffnung (15), die über einen zweiten Strömungskanal (16) für die Exhalation verbunden sind;
einem dem zweiten Strömungskanal zugeordneten ersten Filter (17);
einer Inhalationsmaske (50), in die der erste Strömungskanal (13) endet und an der der zweite Strömungskanal (16) beginnt;
**dadurch gekennzeichnet, dass** die Inhalationsmaske (50) eine dritte Lufteinlassöffnung in Form eines Ventils (63) für einen dritten Strömungskanal aufweist um einen kontinuierlichen Luftdurchfluss zu gewährleisten;
wobei die Exhalation über den zweiten Strömungskanal (16) erfolgt.

2. Bauteil nach Anspruch 1, wobei bei Beendigung der Inhalation der erste Strömungskanal (13) verschlossen, und der zweite Strömungskanal (16) freigegeben wird.

3. Bauteil nach Anspruch 1 oder 2, wobei die erste Luftauslassöffnung (12) und die zweite Lufteinlassöffnung (14) zusammenfallen.

4. Bauteil nach einem der Ansprüche 1 bis 3, wobei der erste Strömungskanal (13) und der zweite Strömungskanal (16) ein Drei-Wege-Ventil bilden.

5. Bauteil nach einem der Ansprüche 1 bis 4, wobei die erste Luftauslassöffnung (12) bzw. die zweite Lufteinlassöffnung (14) mit einem Mundstück (20) verbindbar ist.

6. Bauteil nach Anspruch 5, ferner mit einem Schutzelement (22), das das Mundstück (20) in Umfangsrichtung umgibt und sich über die Mundstückspitze (21) hinaus erstreckt.

7. Bauteil nach Anspruch 6, wobei das Schutzelement (22) an dem der Mundstückspitze (21) gegenüberliegenden Ende dichtend mit dem Mundstück (20) verbunden ist.

8. Bauteil nach einem der Ansprüche 6 oder 7, wobei der Überstand des Schutzelementes (22) gegenüber der Mundstückspitze (21) zwischen 5 und 100 mm beträgt.

9. Bauteil nach einem der Ansprüche 6, 7 oder 8, wobei das Schutzelement (22) selbstexpandierend ist.

10. Bauteil nach einem der Ansprüche 6 bis 9, wobei das Schutzelement (22) als Faltenbalg ausgebildet ist.

11. Bauteil nach einem der Ansprüche 5 bis 10, wobei die Inhalationsmaske (50) eine erste Öffnung (51) aufweist, an der der erste Strömungskanal (13) endet.

12. Bauteil nach Anspruch 11, wobei das Mundstück (20) die Inhalationsmaske (50) durchdringt und sich in den durch die Inhalationsmaske (50) und das Gesicht des Benutzers gebildeten Maskeninnenraum (56) erstreckt.

13. Bauteil nach Anspruch 11 oder 12, wobei das Mundstück (20) verschiebbar zwischen einer Inhalationsstellung, in der der Benutzer das Mundstück (20) in den Mund nehmen kann und einer Exhalationsstellung, in der das Mundstück (20) in den Maskeninnenraum (56) zurückgezogen ist, in der ersten Öffnung (51) angeordnet ist.

14. Bauteil nach einem der Ansprüche 1 bis 13, wobei die Inhalationsmaske (50) im Umfangsbereich (55) derart ausgebildet ist, dass im Falle eines Unterdrucks in dem durch die Inhalationsmaske (50) und dem Gesicht des Benutzers gebildeten Maskeninnenraum (56) ein Druckausgleich über den Umfangsbereich (55) ermöglicht wird.

15. Bauteil nach einem der Ansprüche 1 bis 14, wobei die Inhalationsmaske (50) mindestens ein Einwegventil (60) zum Druckausgleich im Maskeninneren (56) aufweist.

16. Bauteil nach einem der Ansprüche 1 bis 15, wobei die Inhalationsmaske (50) ballonförmig ausgebildet ist.

17. Bauteil nach Anspruch 16, wobei das Mundstück (20) die Inhalationsmaske (50) in einem ersten vom Gesicht des Benutzers abgewandten Bereich (57) durchdringt und sich in den Maskeninnenraum (56) erstreckt und ein gegenüberliegender zweiter, dem Gesicht des Benutzers zugewandter Bereich (58) beim Anlegen der Inhalationsmaske (50) von der Mundstückspitze (21) durchdringbar ist.

18. Bauteil nach einem der Ansprüche 1 bis 17, wobei die zweite Luftauslassöffnung (15) mit einer Absaugeinrichtung (40) verbindbar ist.

19. Bauteil nach einem der Ansprüche 1 bis 18, wobei die erste Lufteinlassöffnung (11) mit einem Vernebler (30) verbindbar ist.

20. Bauteil nach einem der Ansprüche 1 bis 19, wobei das Bauteil Bestandteil des Aerosolgenerators der Inhalationsvorrichtung ist.

21. Inhalationsvorrichtung mit einem Bauteil nach einem der Ansprüche 1 bis 20.

22. Inhalationsvorrichtung nach Anspruch 21, ferner mit einem mit dem ersten Strömungskanal verbundenen Vernebler (30).

23. Inhalationsvorrichtung nach Anspruch 22, ferner mit einer mit dem zweiten Strömungskanal verbundenen Absaugeinrichtung.

24. Inhalationsvorrichtung nach Anspruch 23, wobei die Inhalationsmaske (50) ferner einen Drucksensor (62) aufweist, der mit der Absaugeinrichtung und/oder mit der Inhalationsvorrichtung betrieblich verbunden ist.

## Claims

1. Component for an inhalation device (1) comprising:
a first air inlet opening (11) and a first air outlet opening (12), which are connected via a first flow channel (13) for the inhalation;
a second air inlet opening (14) and a second air outlet opening (15), which are connected via a second flow channel (16) for exhalation;
a first filter (17) assigned to the second flow channel;
an inhalation mask (50) in which the first flow channel (13) ends and at which the second flow channel (16) starts;
**characterised in that** the inhalation mask (50) has a third air inlet opening in the form of a valve (63) for a third flow channel in order to ensure a continuous air flow;
wherein the exhalation takes place via the second flow channel (16).

2. The component according to claim 1, wherein upon termination of the inhalation, the first flow channel (13) is closed and the second flow channel (16) is opened.

3. The component according to claim 1 or 2, wherein the first air outlet opening (12) and the second air inlet opening (14) coincide.

4. The component according to one of claims 1 to 3, wherein the first flow channel (13) and the second flow channel (16) form a three-way valve.

5. The component according to one of claims 1 to 4, wherein the first air outlet opening (12) and/or the second air inlet opening (14) are connectable to a mouthpiece (20).

6. The component according to claim 5, further comprising a protection element (22) circumferentially surrounding the mouthpiece (20) and extending beyond the tip (21) of the mouthpiece.

7. The component according to claim 6, wherein the protection element (22) is sealingly connected to the mouthpiece (20) at the end opposing the mouthpiece tip (21).

8. The component according to one of claims 6 or 7, wherein the excess length of the protection element (22) vis-à-vis the mouthpiece tip (21) is between 5 and 100 mm.

9. The component according to one of claims 6, 7 or 8, wherein the protection element (22) is self-expandable.

10. The component according to one of claims 6 to 9, wherein the protection element (22) is formed as bellows.

11. The component according to one of claims 5 to 10, wherein the inhalation mask (50) has a first opening (51) at which the first flow channel (13) ends.

12. The component according to claim 11, wherein the mouthpiece (20) penetrates the inhalation mask (50) and extends into the interior (56) of the mask formed by the inhalation mask (50) and the face of the user.

13. The component according to claim 11 or 12, wherein the mouthpiece (20) is arranged movably between an inhalation position, where the user can put the mouthpiece (20) into the mouth, and an exhalation position, where the mouthpiece (20) is withdrawn in the interior (56) of the mask, in the first opening (51).

14. The component according to one of claims 1 to 13, wherein the inhalation mask (50) is formed in the circumferential area (55) such that a pressure compensation via the circumferential area (55) is possible if there is a negative pressure in the interior (56) of the mask formed by the inhalation mask (50) and the face of the user.

15. The component according to one of claims 1 to 14, wherein the inhalation mask (50) has at least a one-way valve (60) for pressure compensation in the interior (56) of the mask.

16. The component according to one of claims 1 to 15, wherein the inhalation mask (50) is designed in the form of a balloon.

17. The component according to claim 16, wherein the mouthpiece (20) penetrates the inhalation mask (50) in a first portion (57) turned away from the user's face and extends into the interior (56) of the mask and an opposing second portion (58) turned towards the face of the user can be penetrated by the mouthpiece tip (21) when the inhalation mask (50) is put on.

18. The component according to one of claims 1 to 17, wherein the second air outlet opening (15) is connectable to a suction device (40).

19. The component according to one of claims 1 to 18, wherein the first air inlet opening (11) is connectable to a nebulizer (30).

20. The component according to one of claims 1 to 19, wherein the component is part of the aerosol generator of the inhalation device.

21. An inhalation device comprising a component according to any of claims 1 to 20.

22. The inhalation device according to claim 21, further comprising a nebulizer (30) connected to the first flow channel.

23. The inhalation device according to claim 22, further comprising a suction device connected to the second flow channel.

24. The inhalation device according to claim 23, wherein the inhalation mask (50) further comprises a pressure sensor (62), which is operationally connected to the suction device and/or the inhalation device.

## Revendications

1. Composant pour un dispositif d'inhalation (1) avec ;
une première ouverture de prise d'air (11) et une première ouverture de sortie d'air (12) qui sont reliées via un premier canal d'écoulement (13) pour l'inhalation ;
une deuxième ouverture de prise d'air (14) et une deuxième ouverture de sortie d'air (15) qui sont reliées via un deuxième canal d'écoulement (16) pour l'exhalation ;
un premier filtre (17) affecté au deuxième canal d'écoulement ;
un masque d'inhalation (50), dans lequel se termine le premier canal d'écoulement (13) et au niveau duquel le deuxième canal d'écoulement (16) commence ;
**caractérisé en ce que** le masque d'inhalation (50) comprend une troisième ouverture de prise d'air sous la forme de valve (63) pour un troisième canal d'écoulement pour garantir un écoulement d'air continu ;
sachant que l'exhalation s'effectue par le deuxième canal d'écoulement (16).

2. Composant selon la revendication 1, dans lequel, à la fin de l'inhalation, le premier canal d'écoulement (13) est verrouillé, et le deuxième canal d'écoulement (16) est débloqué.

3. Composant selon la revendication 1 ou 2, dans lequel la première ouverture de sortie d'air (12) et la deuxième ouverture de prise d'air (14) se rejoignent.

4. Composant selon l'une des revendications 1 à 3, dans lequel le premier canal d'écoulement (13) et le deuxième canal d'écoulement (16) forment une valve à trois voies.

5. Composant selon l'une des revendications 1 à 4, dans lequel la première ouverture de sortie d'air (12) et/ou la deuxième ouverture de prise d'air (14) peut être raccordée à un embout (20).

6. Composant selon la revendication 5, en outre avec un composant de protection (22) qui entoure l'embout (20) dans une direction circonférentielle et s'étend vers l'extérieur sur la pointe de l'embout (21).

7. Composant selon la revendication 6, dans lequel l'élément de protection (22) est relié de manière hermétique à l'embout (20) au niveau de l'extrémité opposée de la pointe de l'embout (21).

8. Composant selon l'une des revendications 6 ou 7, dans lequel la projection de l'élément de protection (22) par rapport à la pointe de l'embout (21) mesure entre 5 et 100 mm.

9. Composant selon l'une des revendications 6, 7 ou 8, dans lequel l'élément de protection (22) est auto-expansible.

10. Composant selon l'une des revendications 6 à 9, dans lequel l'élément de protection (22) est conçu comme un soufflet plissé,

11. Composant selon l'une des revendications 5 à 10, dans lequel le masque d'inhalation (50) comprend une première ouverture (51), au niveau de laquelle se termine le premier canal d'écoulement (13).

12. Composant selon la revendication 11, dans lequel l'embout (20) pénètre le masque d'inhalation (50) et se prolonge dans l'espace intérieur du masque (56) formé par le masque d'inhalation (50) et le visage de l'utilisateur.

13. Composant selon la revendication 11 ou 12, dans lequel l'embout (20) est disposé dans la première ouverture (51) de manière déplaçable entre une position d'inhalation dans laquelle l'utilisateur peut prendre l'embout (20) dans sa bouche et une position d'exhalation dans laquelle l'embout (20) peut être rétracté dans l'espace intérieur du masque (56).

14. Composant selon l'une des revendications 1 à 13, dans lequel le masque d'inhalation (50) est formé dans le secteur périphérique (55) de telle sorte qu'en cas de dépression dans l'espace intérieur du masque (56) formé par le masque d'inhalation (50) et le visage de l'utilisateur, une compensation des pressions est possible sur le secteur périphérique (55).

15. Composant selon l'une des revendications 1 à 14, dans lequel le masque d'inhalation (50) comprend au moins une valve anti-retour (60) pour une compensation des pressions à l'intérieur du masque (56).

16. Composant selon l'une des revendications 1 à 15, dans lequel le masque d'inhalation (50) est conçu en forme de ballon.

17. Composant selon la revendication 16, dans lequel l'embout (20) pénètre le masque d'inhalation (50) dans un premier secteur (57) opposé au visage de l'utilisateur et s'étend dans l'espace l'intérieur du masque (56) et un deuxième secteur (58) opposé tourné vers le visage de l'utilisateur peut être pénétré par la pointe de l'embout (21) lors de l'application du masque d'inhalation (50).

18. Composant selon l'une des revendications 1 à 17, dans lequel la deuxième ouverture de sortie d'air (15) peut être raccordée à un dispositif d'aspiration (40).

19. Composant selon l'une des revendications 1 à 18, dans lequel la première ouverture de prise d'air (11) peut être raccordée à un nébuliseur (30).

20. Composant selon l'une des revendications 1 à 19, dans lequel le composant fait partie du générateur d'aérosol du dispositif d'inhalation.

21. Dispositif d'inhalation avec un composant selon l'une des revendications 1 à 20.

22. Dispositif d'inhalation selon la revendication 21 comprenant en outre un nébuliseur (30) relié au premier canal d'écoulement.

23. Dispositif d'inhalation selon la revendication 22 comprenant en outre un dispositif d'aspiration relié au deuxième canal d'écoulement.

24. Dispositif d'inhalation selon la revendication 23, dans lequel le masque d'inhalation (50) comprend en outre un capteur de pression (62) qui est relié, lors de son utilisation, au dispositif d'aspiration et/ou au dispositif d'inhalation.
